# EUROPEAN PATENT SPECIFICATION

(11) **EP 1 092 438 B1**
(45) Date of publication and mention of the grant of the patent: **14.06.2006**
(21) Application number: 00108770.9
(22) Date of filing: 25.04.2000
(51) Int. Cl.: A61K 38/31, A61P 35/00

(54) **Pharmaceutical compositions containing growth-hormone inhibitors or their biologically active fragments for the treatment of uterine myomas**
Wachstumhormone Inhibitoren Enthaltende pharmazeutische Zubereitungen oder ihre biologisch aktive Fragmente zur Behandlung von uterinen Myomas
Compositions pharmaceutiques contenant des Inhibiteurs de l'hormone de croissance ou leurs fragments biologiquement actifs pour le traitement de myomes utérins

(30) Priority: 27.04.1999 IT FI990097
(43) Date of publication of application: 18.04.2001
(73) Proprietor: De Leo, Vincenzo, 53100 Siena (IT); Interbiotek S.r.l., 56010 Vico Pisano (Pisa) (IT)
(72) Inventor: De Leo, Vincenzo, 53100, Siena (IT)
(74) Representative: Gervasi, Gemma

(56) References cited:
- US-A- 3 904 594
- G. WECKBECKER ET AL.: "SOMATOSTATIN ANALOGUE OCTREOTIDE ENHANCES THE ANTINEOPLASIC EFFECTS OF TAMOXIFEN AND OVARIECTOMY ON 7,12-DIMETHYLBENZ(A)ANTHRACENE-INDUCED RAT MAMMARY CARCINOMAS" CANCER RESEARCH, vol. 54, no. 24, 15 December 1994 (1994-12-15), pages 6334-6337, XP002122190 BALTIMORE, MD, US

## Description

### Field of invention

The present invention refers to pharmaceutical compositions containing growth-hormone inhibitors or their biologically active fragments for the treatment of uterine myomas.

### State of the art

Uterine myomas, in particular leiomyomas or fibromas, are the benign tumours most commonly occurring in women. They occur in approximately 30% of women aged over 35 in the form of a thickening of the wall of the uterus that may reach even considerable dimensions, and are frequently associated to infertility and to clinical signs, such as menorrhagia and dysmenorrhea.

The presence of leiomyomas often calls for surgical intervention whereby removal of the myoma alone is performed in the case of young women, whilst for women aged over 40 total hysterectomy is preferred.

Even though the surgical operation does not present particular difficulties from the clinical point of view, it is nonetheless delicate and involves certain problems. In particular, the normal anatomy of the pelvis is completely modified, and this may in some cases be accompanied by disorders of normal physiological functions of the genito-urinary apparatus.

So far the most effective medical therapy has been represented by GnRH analogues, which, through a blockage of the hypothalamus-hypophysis-gonads axis induce a state of pharmacologically induced menopause with hypo-oestrogenism.

At the end of the therapy, the fibromas generally return to the starting conditions in relatively short periods of time, and in any case always within a range of a few months. In addition, one of the main side effects of the treatment is represented by the high incidence of menopausal symptoms, which considerably reduces patient compliance.

In the light of what has been said above, the importance is evident of having available a drug that is capable of solving the myoma, or at least causing it to regress, without the need for performing surgery, or putting surgery off to when ihe woman is older, without inducing disagreeable side effects.

On the other hand, it is known that growth-hormone inhibitors, such as somatostatin and its analogues or their biologically active fragments, are indicated in the treatment of acromegaly, clinical signs of certain carcinoid tumours, especially gastro-enteropancreatic ones, in postoperative complications following on interventions on the pancreas, in pancreatic fistulas, and in GH-secreting tumours of the hypophysis.

In G.Weckbecker et al. Cancer Research Vol. 54, n° 24, 15 December 1994, pp. 6334-37 it is reported that octreotide enhances the antineoplastic effect of Tamoxifen and Ovariectomy on 7,12-dimethylbenz(a)anthracene-induced rat mammary carcinomas.

US - 3,904,594 describes various peptides which inhibit the secretion of growth hormone, useful in the treatment of acromegaly and diabetes and in controlling the release of thyroid stimulating hormone.

### Detailed description of invention

It has now been surprisingly found that pharmaceutical compositions containing growth-hormone inhibitors or their biologically active fragments have an effect of reducing myomas, an effect that can even arrive at a total regression of the myoma in an extremely limited period of time.

Among the growth-hormone inhibitors according to the invention, we recall in particular somatostatin or its analogues and their biologically active fragments.

More in particular, the following products have proved of interest:
Somatostatin-28, Tyr-Somatostatin-28, (Leu⁸, D-Trp²², Tyr²⁵)-Somatostatin-28, Biotinyl-(Leu⁸, D-Trp²² , Tyr²⁵)-Somatostatin-28, Somatostatin-28(1-14), (Tyr¹²)-Somatostatin-28(1-14), Somatostatin-28(1-12), Somatostatin-25, Somatostatin-14, Somatostatin-14 acetate hydrate, Somatostatin-14 diacetate hydrate, Somatostatin-14 (coupled to BSA), Somatostatin-14 (coupled to KLH), Tyr-Somatostatin-14, (D-Trp⁸)-Somatostatin-14, (D-Trp⁸, D-Cys¹⁴)-Somatostatin-14, (Tyr¹)-Somatostatin-14, (Tyr¹¹)-Somatostatin-14, (des-Ala¹, des-Gly², His^{4.5}, D-Trp⁸)-Somatostatin-14, [D-Trp⁸, Tyr¹¹]-Somatostatin, Biotinyl-[D-Trp⁸, Tyr¹¹]-Somatostatin, [D-Trp¹¹]-Somatostatin, Octreotide, and Lanreotide.

The pharmaceutical compositions containing the above-mentioned active principles useful for the purpose indicated by the invention can be formulated according to usual methods described in pharmacopoeia according to the type of administration preferred. The formulations may be in the fast-release form or in the long-acting, i.e., slow-release form, useful for spreading out the administrations and improving patient compliance, as well as providing therapeutic coverage of the patient. Long-acting forms of drugs are also referred to as depot forms.

The preferred compositions are the ones to be administered by intramuscular or subcutaneous injection or by intravenous infusion, prepared both in the form of ampoules ready for use and as a lyophilized composition to be re-dissolved at the moment of use (vials).

The excipients normally used for the preparation of the solution in the case of injectable ampoules are, in addition, obviously, water for injections (w.f.i.), lacticoglycol copolymer, mannitol, carboxymethylcellulose Na, polysorbate, etc.

The vials for dilution of the lyophilized compound normally contain: w.f.i., sodium chloride, anhydrous monosodium phosphate, disodium phosphate dodecahydrate, etc.

The active principle is normally between 250 µg and 30 mg.

In what follows a number of examples of formulations are given:
Active principle: lanreotide 0.030 g
Excipients: (lactide-glycolide) copolymer, (lactic-glycolic) copolymer, mannitol, carboxymethylcellulose Na, polysorbate 80, w.f.i..
Active principle: octreotide 0.05 - 0.01 ; 0.2 - 0.5 mg/ml
Excipients: (vials) lactic acid, mannitol, sodium bicarbonate, w.f.i.; (ampoules) lactic acid, phenol, mannitol, sodium bicarbonate, w.f.i..
Active principle: octreotide acetate corresponding to octreotide as free peptide 10-20-30 mg.
Excipients: Sterile poly DL-lactide-coglycolide mannitol.
Active principle: octreotide (vials) 0.05 - 0.01 - 0.5 mg; (ampoules) 0.2 mg/ml.
Excipients: (vials) lactic acid, mannitol, sodium bicarbonate, w.f.i.; (ampoules) lactic acid, phenol, mannitol, sodium bicarbonate, w.f.i..
Active principle: Somatostatin acetate hydrate 250 µg.
Excipients: lyophilized ampoules - mannite; solvent-containing vials - sodium chloride, w.f.i..
Active principle: Somatostatin acetate hydrate equivalent to base somatostatin 250-3000 µg.
Excipients: lyophilized ampoules - mannite; solvent-containing vials - sodium chloride, w.f.i..
Active principle (lyophilized ampoules): Injectable somatostatin acetate 0.25-2 mg.
Excipients (solvent vials): sodium chloride, anhydrous monosodium phosphate. disodium phosphate dodecahydrate, w.f.i..

For the treatment of uterine myomas, amounts of derivatives of somatostatin are administered to the patient in depot form in doses of 30-60 mg starting from the second day of the menstrual cycle, every two weeks for 3-6 months; if fast-release forms of administration are used, the dosage is from 0.1 to 30 mg per day starting from the second day of the menstrual cycle, for 3-6 months.

### Experimental tests

The case was considered of a 44-year-old woman who had already had two pregnancies and who presented menorrhagic cycles every 24-26 days (duration 8-9 days) and a fibromatous uterus having the dimensions of 98 x 56 x 67 mm. In addition, an intramural myoma of 36 mm in the posterior part and a submucous myoma of 34 mm on the anterolateral wall were present. The blood plasma concentrations of FSH and E2 measured in the follicular phase indicated a state of fertility: FSH 12 mlU/ml, E2 80 pg/ml.

Lanreotide was administered on the second day of the cycle, and this administration was repeated every 14 days for 3 months. An ultrasound examination was carried out before the therapy, after each month of therapy, and 3 months after the end of the therapy.

A reduction of 50% in the duration of menstrual bleeding was found, as well as a reduction in quantity of blood to levels equal to those prior to onset of the myomas. Ultrasound examination revealed a reduction of 12% in the uterine volume after one month of treatment and 28% after three months, calculated using the formula of the volume of the sphere.

Normal menstrual flow remained unvaried for the entire application of the therapy and also throughout the three months following on interruption of treatment. Hormone assays performed one month after the start of therapy showed the same levels of FSH and E2 as those already measured before start of therapy, as well as the inhibition of plasma concentrations of GH and IGF-I; the corresponding data are summed up in Table 1. The routine blood parameters remained unvaried before and after treatment.

**TABLE 1 Example 1 (44-year-old patient)**

| | Basal | 3rd month of treatment | Follow-up |
|---|---|---|---|
| Dimens. of uterus (mm)* | 98 × 56 × 67 | 91 × 49 × 62 | 95 × 55 × 60 |
| (cm³)** | 208 | 150 | 179 |
| Myomas (mm)*** | 36; 34 | 30; 29 | 33; 31 |
| (cm³)** | 24; 21 | 14; 13 | 18; 16 |
| FSH (mlU/ml) | 12 | 10.5 | 10 |
| E2 (pglml) | 80 | 72 | 74 |
| GH (ng/ml) | 2.2 | 0.5 | 1.5 |
| IGF-I (IU/ml) | 3.6 | 0.8 | 2.9 |

| | | | |
|---|---|---|---|
| * diameters; ** volume; *** mean diameter | | | |

Treatments conducted on other subjects yielded results likewise favourable, or even better. Data corresponding to two further cases are given in Tables 2 and 3.

**TABLE 2 Example 2 (52-year-old patient)**

| | Basal | 3rd month of treatment | Follow-up |
|---|---|---|---|
| Dimens. of uterus (mm)* | 81 × 53 × 40 | 74 × 46 × 39 | 76 × 44 × 32 |
| (cm³)** | 102 | 78 | 68 |
| Myomas (mm)*** | 12; 27; 30 | 8; 17; 19 | 8; 17; 19 |
| (cm³)** | 0.9; 10; 14 | 0.3; 2.6; 3.6 | 0.3; 2.6; 3.6 |
| FSH (mlU/ml) | 22 | 79 | 84 |
| E2 (pg/ml) | 74 | < 20 | < 20 |
| GH (ng/ml) | 1.3 | 0.1 | 0.8 |
| IGF-I (IU/ml) | 4.1 | 0.8 | 1.3 |

| | | | |
|---|---|---|---|
| * diameters; ** volume; *** mean diameter | | | |

**TABLE 3 Example 3 (49-year-old patient)**

| | Basal | 3rd month of treatment | Follow-up |
|---|---|---|---|
| Dimens. of uterus (mm)* | 118 × 98 × 57 | 95 × 90 × 47 | 94 ×90 × 44 |
| (cm³)** | 394 | 239 | 230 |
| Myomas (mm)*** | 42; 46 | 28; 30 | 27; 30 |
| (cm³)** | 38.7; 51 | 11.5; 14.1 | 10,2; 14,1 |
| FSH (mlU/ml) | 134 | 113 | 120 |
| E2 (pg/ml) | 18 | 15 | 15 |
| GH (ng/ml) | 1.6 | 0.2 | 0.6 |
| IGF-I (IU/ml) | 3.2 | 0.4 | 0.9 |

| | | | |
|---|---|---|---|
| * diameters; ** volume; *** mean diameter | | | |

The data gathered show that somatostatin-analogue derivatives represent a new therapeutic means for the treatment of myomas.

It is interesting to note that the three cases described above each represent a patient of every period of the reproductive life of a woman: the fertile period, the perimenopausal period, and the postmenopausal period.

An original and complete approach of this sort proves that the subject of the invention is certainly endowed with therapeutic efficacy.

## Claims

1. Use of somatostatin, its derivatives, lanreotide octreotide and their biologically active fragments for the preparation of pharmaceutical compositions for the treatment of uterin myomas.

2. Use according to Claim 1, wherein the somatostatin derivatives are: Somatostatin-28, Tyr-Somatostatin-28, (Leu⁸, D-Trp²², Tyr²⁵)-Somatostatin-28, Biotinyl-(Leu⁸, D-Trp²² , Tyr²⁵)-Somatostatin-28, Somatostatin-28(1-14), (Tyr¹²)-Somatostatin-28(1-14), Somatostatin-28(1-12), Somatostatin-25, Somatostatin-14, Somatostatin-14 acetate hydrate, Somatostatin-14 diacetate hydrate, Somatostatin-14 (coupled to BSA), Somatostatin-14 (coupled to KLH), Tyr-Somatostatin-14, (D-Trp⁸)-Somatostatin-14, (D-Trp⁸, D-Cys¹⁴)-Somatostatin-14, (Tyr¹)-Somatostatin-14, (Tyr¹¹)-Somatostatin-14, (des-Ala¹, des-Gly², His^{4,5}, D-Trp⁸)-Somatostatin-14, [D-Trp⁸, Tyr¹¹]-Somatostatin, Biotinyl-[D-Trp⁸, Tyr¹¹]-Somatostatin, [D-Trp¹¹]-Somatostatin.

3. Use according to claims 1 - 2 wherein the pharmaceutical composition is in the form to be administered by intramuscular or subcutaneous injection or by intravenous infusion.

4. Use according to claims 1 - 3 wherein the pharmaceutical composition contains the active principle in quantities of between 250 µg and 30 mg.

## Patentansprüche

1. Verwendung von Somatostatin, seinen Derivaten, Lanreotid, Octreotid und ihren biologisch aktiven Fragmenten für die Herstellung von pharmazeutischen Zusammensetzungen für die Behandlung von Uterusmyomen.

2. Verwendung gemäß Anspruch 1, wobei die Somatostatinderivate die folgenden sind:
Somatostatin-28, Tyr-Somatostatin-28, Leu⁸, D-Trp²², Tyr²⁵)-Somatostatin-28, Biotinyl-(Leu^{B}, D-Trp²², Tyr²⁵)-Somatostatin-28, Somatostatin-28 (1-14), (Tyr¹²)-Somatostatin-28(1-14), Somatostatin-28(1-12), Somatostatin-25, Somatostatin-14, Somatostatin-14-acetathydrat, Somatostatin-14-diacetathydrat, Somatostatin-14 (gekoppelt an BSA), Somatostatin-14 (gekoppelt an KLH), Tyr-Somatostatin-14, (D-Trp⁸)-Somatostatin-14, (D-Trp⁸, D-Cys¹⁴)-Somatostatin-14, (Tyr¹)-Somatostatin-14, (Tyr¹¹)-Somatostatin-14, (des-Ala¹, des-Gly², His^{4,5}, D-Trp⁸)-Somatostatin-14, [D-Trp⁸, Tyr¹¹]-Somatostatin, Biotinyl-[D-Trp⁸, Tyr¹¹]-Somatostatin und [D-Trp¹¹]-Somatostatin.

3. Verwendung gemäß einem der Ansprüche 1 bis 2, wobei die pharmazeutische Zusammensetzung sich in einer Form befindet, die durch intramuskuläre oder subkutane Injektion oder intravenöse Infusion zu verabreichen ist.

4. Verwendung gemäß einem der Ansprüche 1 bis 3, wobei die pharmazeutische Zusammensetzung den Wirkstoff in Mengen von 250µg bis 30 mg enthält.

## Revendications

1. Utilisation de la somatostatine, de ses dérivés, du lanréotide, de l'octréotide et de leurs fragments biologiquement actifs pour la préparation de compositions pharmaceutiques destinées au traitement de myomes utérins.

2. Utilisation selon la revendication 1, dans laquelle les dérivés de la somatostatine sont : la somatostatine 28, la Tyr-somatostatine 28, la (Leu⁸, D-Trp²², Tyr²⁵) - somatostatine 28, la biotinyl-(Leu⁸, D-Trp²², Tyr²⁵) - somatostatine 28, la somatostatine 28 (1-14), la (Tyr¹²)- somatostatine 28 (1-14), la somatostatine 28 (1-12), la somatostatine 25, la somatostatine 14, l'acétate de somatostatine 14 hydraté, le diacétate de somatostatine 14 hydraté, la somatostatine 14 (couplée à la SAB), la somatostatine 14 (couplée à la KLH), la Tyr-somatostatine 14, la (D-Trp⁸)-somatostatine 14, la (D-Trp⁸, D-Cys¹⁴)- somatostatine 14, la (Tyr¹-somatostatine 14, la (Tyr¹¹) - somatostatine 14, la (dés-Ala¹, dés-Gly², His^{4,5}, D-Trp^{B}) -somatostatine 14, la [D-Trp⁸, Tyr¹¹] -somatostatine, la biotinyl-[D-Trp⁸, Tyr¹¹] -somatostatine, la [D-Trp¹¹]-somatostatine.

3. Utilisation selon l'une quelconque des revendications 1 à 2, dans laquelle la composition pharmaceutique se présente sous une forme destinée à être administrée par injection intramusculaire ou sous-cutanée ou par perfusion intraveineuse.

4. Utilisation selon l'une quelconque des revendications 1 à 3, dans laquelle la composition pharmaceutique contient le principe actif en des quantités allant de 250 µg à 30 mg.
